# EUROPEAN PATENT APPLICATION

(11) **EP 4 632 376 A1**
(43) Date of publication of application: **15.10.2025**
(21) Application number: 24183641.0
(22) Date of filing: 21.06.2024
(51) Int. Cl.: G01N 33/205

(54) **METHOD AND SYSTEM FOR DETERMINING A PARAMETER OF A HIGH TEMPERATURE LIQUID**

(30) Priority: 12.04.2024 EP 24169906; 12.06.2024 EP 24181621; 12.06.2024 EP 24181625
(71) Applicant: Heraeus Electro-Nite International N.V., 3530 Houthalen (BE)
(72) Inventor: Neyens, Guido, 3530 Houthalen (BE); Bex, Gert-Jan, 3530 Houthalen (BE); Stuer, Giovanni, 3530 Houthalen (BE); Vromans, Martijn, 3530 Houthalen (BE)
(74) Representative: Heraeus IP

(57) **Abstract**

The present invention relates to a method for determining at least one parameter of a high temperature liquid with a sensor unit and a system to carry out the method. A measuring probe releasably carrying the sensor unit is provided to acceleration means, which accelerates the sensor unit after separation from the measuring probe. The acceleration means is provided in a distance D_{A} to the surface of the high temperature liquid and the distance D_{A} between the surface of the high temperature liquid is less than 50 % of the distance of the surface of the high temperature liquid to the opening of the container D_{M}. The sensor unit is projected in the direction of the high temperature liquid, immersed under the surface and the parameter of interest is measured. The invention further relates to a system suitable to carry out the inventive method and a metallurgical vessel comprising the inventive system.

## Description

The present invention relates to a method for determining at least one parameter of a high temperature liquid with a sensor unit and a system to carry out the method. The invention further relates to a metallurgical vessel comprising the inventive system.

Especially during the metal making process employed in the steel industry, several parameters of the metal melt are critical for the control of the metallurgical process, for example the bath chemistry or the temperature of such a melt. The ability to continuously and/or periodically monitor these variables is highly desirable for both economic and quality reasons. Accurate monitoring can for once greatly reduce energy consumption caused by overheating. Other benefits of a continuous monitoring include the ability to measure high temperature phase changes, chemical reactions, and other related phenomena.

Methods and devices to determine these process relevant parameters are known in the field, often involving at least the use of a disposable probe carrying a sensor. Typically, the probe is brought under the surface of the melt in form of a drop-in unit or by means of a lance assembly. The lance assembly can be operated manually, fully or semi-automated. The sensor is connected with a processing device for processing the recorded data, typically by wires or cables, but also wireless data transfer has been described. Data is gathered and processed at or near real-time and provide the operator of the metallurgical facility with critical information about the progress or status of the metal making process occurring in the vessel.

Lance-assemblies to introduce a probe into a molten metal require a number of parts, at least a measuring head carrying the disposable sensor and the lance itself. The lance is dipped from an opening in the vessel, i.e. in position close to the molten metal, into the melt, which causes splashing of the metal and is especially in cases of a manual operation a dangerous operation. For the introduction of such a lance into the molten metal, the operation of the metallurgical vessel has to be interrupted to open the vessel which lowers the process yield. Depending on the operating personnel used, the measuring quality varies. The accuracy of the measurement depends on several parameters like the immersion depth and the immersion speed of the probe into the liquid metal melt. Too slow immersion leads to premature burning of the probe and to a false measurement. If the probe is not immersed deep enough, the temperature may be unstable, or the electromotive force of an oxygen sensor may become unreliable. The manual operation of such a lance is additionally undesired from a safety point of view - the general trend in the industry is to have a "manless" operation as much as possible.

Automatically operated devices solve this problem but require a higher amount of operating equipment and maintenance. The operation of such devices is also based on the insertion of the probe through a relatively large opening. Additionally, all installations next to the vessel are prone to heat or mechanical damages and the required opening must be kept free of blockages.

Furthermore, a new probe has to be fitted on a lance after every measurement, as for example described in WO 2015070316 A1, which may require further components, process steps and lengthens the time interval between subsequent measurements. A higher measurement frequency is thus desirable.

Especially in the field of electric arc furnaces (EAFs), there are presently only limited methods available to determine the parameters of the molten metal which can be conducted during the operation of the vessel. EAFs produce steel by using an electric arc to melt one or more charges of scrap metal, hot metal, iron based materials, or other meltable materials, which are placed within the furnace. In procedures common in EAFs, an operator manually inserts a lance carrying a suitable sensor into the furnace through the slag door, which is a relatively wide opening in the wall of the furnace shell. When the slag door is opened, any slag and metal trapped at the door opening must be cleared to allow insertion of the measurement probe. Such an invasive step is highly undesirable since it disturbs the environment within the vessel during the metallurgical process. Furthermore, energy is wasted caused by the intake of cold ambient air through the slag door when it is opened.

For measurements with drop-in sensors the measuring probe is dropped into the melt containing vessel. Suitable probes are for example disclosed in EP 0758445 A1. In current practice, the sensors are introduced from a position above the vessel containing the molten metal from a relatively large height, typical in the range of 10 to 20 m above the level of the molten metal. Due to the design of an EAF with the electrodes positioned above the vessel, such sensors cannot be applied in these facilities.

Several probes can be stored in a magazine and one probe at a time is released from the magazine for each measurement. The probe falls in free fall, accelerated by gravity, and dives into the molten metal. The final speed of the probe when arriving at the molten metal surface is therefore determined by the distance between the drop station and the molten metal. The probes need to have a certain mass in order to dive deep enough under the melt surface to obtain reliable measurement data. Further components to provide a required orientation of the sensor, like balancing bodies, which are not related to the recording of the measurement itself are required in order to provide reliable data. Therefore, drop-in sensors introduce a relatively large amount of extra contaminating material into the to be measured molten material. Furthermore, the immersion point is not reliably controllable.

A molten metal is typically covered with a slag layer during its production, hereby subjecting any probe or sensor passing through it to increased erosive conditions, regardless of the specific methodology utilized. Thus, it is desired to minimize the duration of exposure of the sensor to the slag as much as possible.

Known injection devices as applied in the metallurgical field are used for the introduction of liquid and/or particle-shaped material for the pyrometallurgical treatment of metal melts. In particular in EAFs such injectors are utilized for blowing oxygen-rich gases, lime and/or carbon-containing particles into the metallurgical vessel. Typically, these devices are positioned near to the surface of the to be treated liquid metal through an appropriate opening in the vessel. Often, such injectors are provided as a combined unit sitting in a so-called cold box, which is a protecting compartment provided adjacent to the interior of the vessel. All openings are kept clean of clogging during operation, usually by a flow of nitrogen, or compressed air flow. Further openings are thus not desired, since the increased demand of gas would increase the cost of operations. Therefore, it would be advantageous to provide a method which utilizes available openings further.

In view of the prior art, there is the need for an improved measurement method and system to allow a non-invasive measurement without or with only minimal (human) intervention, the insertion of a lance or comparable invasive actions. Furthermore, there is the requirement for a fast and reliable method, which can be conducted at a high frequency, and which delivers reproducible results. Additionally, the mass of material introduced by the measurement shall be minimized.

The objective of the invention is thus to provide an improved method for determining at least one parameter of a high temperature liquid like a molten metal with a sensor unit which solves at least one of the problems discussed above.

In particular, one of the objectives is to provide an improved method which allows the use of a simplified sensor unit with reduced weight and number of components. Furthermore, it is an objective to provide a method to obtain the parameter when the sensor unit is in a certain immersion depth under the surface of the high temperature liquid.

An additional aspect of the objectives of the present invention is to provide a method which allows a simplification of the hardware required to carry out the method. Furthermore, available entrance-points in the container with the high temperature liquid shall be utilized to introduce the sensor unit into the container containing the high temperature liquid.

A further objective is to reduce the exposure time of the sensor unit to the environment in the container prior to the immersion into the high temperature liquid as much as possible.

A further objective of the invention is to provide a system configured to carry out the inventive method.

In a further objective of the invention is to provide a system for implementing the inventive method, which allows the determination of the parameters with reduced effort and expenses in terms of equipment, control technology, and organization, while at the same time achieving increased reliability and quality of the obtained measurements.

A further objective of the invention is to provide a metallurgical vessel comprising a system configured to carry out the inventive method.

A further objective of the invention is to provide a device comprising acceleration means configured to carry out the inventive method.

These objectives are attained by the subject-matter defined in the independent claims.

The invention provides a method for determining at least one parameter of a high temperature liquid with a sensor unit,
wherein the high temperature liquid comprises a surface and is provided in a metallurgical container which comprises a top opening opposite the surface of the high temperature liquid, and the surface of the high temperature liquid level has a position L_{M}, which is positioned in a distance D_{M} to the top opening of the metallurgical container, the method comprising
(a) providing acceleration means above the surface of the high temperature liquid in a distance D_{A} between the surface of the high temperature liquid and the top opening of the metallurgical container, wherein the acceleration means is adapted to increase the speed of the sensor unit;
(b) providing a measuring probe to the acceleration means, wherein the measuring probe carries the sensor unit and wherein the sensor unit is separable from the measuring probe;
(c) separating the sensor unit from the measuring probe;
(d) accelerating the sensor unit with the acceleration means;
(e) projecting the sensor unit in the direction of the high temperature liquid;
(f) immersing the sensor unit under the surface of the high temperature liquid;
(g) measuring the at least one parameter of the high temperature liquid;
characterized in that D_{A} < 50 % D_{M}.

The steps of the method are conducted in the given consecutive order.

Surprisingly, it has been found that an acceleration of the sensor unit by more than only gravity offers several advantages. The probe accommodating the sensor unit can be reduced in terms of weight, dimensions and number of components. The reduced material demand results in a cost reduction and a reduction of contaminating material introduced into the high temperature liquid. This miniaturization of the sensor and the related carrier probe in turn allows to introduce the sensor unit through openings with a reduced size which were previously not usable for the introduction of probes with state of the art methods.

Additionally, the acceleration means is placed in a shorter distance to the high temperature liquid, which minimizes the exposure time of the sensor unit to the environment of the container even further. In metallurgical facilities, entry points to treat the molten metal are typically placed in a short distance to the metal surface and were not considered as suitable as points of access for measurement devices due to their demanding environment. These entry points can be used with a double function by the inventive method, i.e. for the introduction of treatment material as well as the sensor unit. Especially in containers where no access from the top side is available for the introduction of measurement equipment, the inventive method expands the possibilities to obtain reliable data.

Another advantage of the inventive method is that it is to be carried out entirely without the use of a lance, which further reduces the demand of components like drive or control means for operation. Thus, the method can be applied in almost all metallurgical vessels. Additionally, the need for human interaction is minimized, enhancing the safety of operation. Especially in EAFs, this offers the further advantage that an opening of the slag-door is not needed to obtain a measurement. Furthermore, it is possible to minimize the interruptions of a metallurgical facility for obtaining required parameter(s), in particular the method is applicable during a continuous operation. This minimizes the total operating costs, in particular the required energy input, and increases the throughput of the metallurgical facility as well as the quality of the products produced.

A further factor reducing the demands on the system in terms of set up and maintenance is a simplified operating system, which can be positioned in a short distance to the vessel containing the high temperature liquid.

Furthermore, the method allows a reproducible impact area of the sensor unit on and into the surface of the high temperature liquid due to a controlled angle and speed of immersion.

The invention relates to the determination of a parameter of a high temperature liquid. A high temperature liquid is to be understood as a liquid with a temperature above 600 °C, preferably above 800 °C, more preferably above 1000 °C. The temperature of the high temperature liquid can for example lie in the range of 1000 - 1900 °C, more preferably in the range of 1200 - 1800 °C, even more preferred in the range of 1400 - 1700 °C.

The nature of the high temperature liquid is not further restricted, preferably the high temperature liquid is a melt of a material with a melting point above 500 °C, in particular a melt of a metal, a cryolite or a glass. Preferably, the high temperature liquid is a molten metal, most preferably a molten steel. The term "melt" or "molten metal" does not exclude the presence of any solid or gaseous parts, including for example non-molten parts of the respective metal. The temperature of metal melts differs and usually depends on the composition of the metal and the stage of the melting process.

In case of a metal melt, the melt may be covered with a slag layer. The term "slag" refers to non-steel byproducts that are often produced in a steel making furnace and are typically present as a molten material that floats on top of the molten metal. Slag may comprise metal oxides, metal sulfides, calcium oxide, magnesium oxide, magnesite, dolomite, iron oxide, aluminum oxide, manganese oxide, silica, sulfur, phosphorous, or a combination thereof. In order to obtain reliable measurements, a sensor introduced into the melt should pass through the slag layer as fast as possible to minimize corrosive effects prior to reaching the final point of measurement and the freezing of the slag material on the cold sensor. Such a frozen layer needs to melt when the sensor finally reaches the molten metal before a reliable measurement can be taken, thus prolonging the time the sensor needs to withstand the decomposing environment of the molten metal.

The high temperature liquid is provided in a metallurgical container, in other words the high temperature liquid is provided in a suitable vessel in form of a bath. The high temperature liquid comprises a surface, accordingly, the high temperature liquid also comprises a bottom side. The bottom side is to be understood as the side of the bath of the high temperature liquid which is in contact with the interior of the metallurgical container opposite to the surface.

The metallurgical container may be any container suitable to accommodate the high temperature liquid, it may for example be a metal treatment vessel or a furnace, in particular an electric arc furnace. In preferred embodiments, the metallurgical container is an electric arc furnace.

The metallurgical container comprises a bottom and a top opening opposite the bottom. The bottom is to be understood of the part of the metallurgical container which is at least partly in contact with the high temperature liquid, in particular with the bottom side of the high temperature liquid bath. The surface of the high temperature liquid level has a position L_{M} in a distance D_{M} to the top opening of the metallurgical container.

Preferably, the metallurgical container comprises installations fixedly mounted on or at it. Such installations can for example be means for heating, as electrodes, means for measurements or means for treating the high temperature liquid. In case of molten metals, such means for treating the molten metal can for example be carbon injectors, lime injectors, oxygen blowing lances, oxy-fuel-lances or air-fuel-burners.

The metallurgical container typically comprises an interior surface defining an interior volume, wherein the interior volume is adapted to contain the high temperature liquid, for example a bath of molten metal or molten glass. As used herein, in particular the term "molten metal bath" is used to describe a metal melt in the metallurgical container. The interior surface of the metallurgical container may include the bottom and at least one sidewall or a plurality of sidewalls.

Typically, such a metallurgical container also comprises at least one entry point, through which the high temperature liquid, preferably a molten metal bath, can be accessed and/or treated. Such an entry point may be positioned in a side wall. In cases of EAFs, multiple openings can be available: a larger opening, typically named slag door and entry points positioned in the side wall.

The invention provides a method for determining a parameter of the high temperature liquid. The parameter can be a physical, chemical or metallurgical parameter, for example the temperature, the presence and/or concentration of a chemical compound, in particular the oxygen content, the carbon content, the hydrogen content, the nitrogen content, the aluminum content or the chemical composition.

"Determining a parameter" may be used herein as a synonym for measuring a parameter. According to a preferred embodiment, the parameter can be determined from a single point measurement or a multiple point measurement. The determination can comprise the determination of a single parameter or the combination of more than one parameter. For example, the determination can comprise the measurement of the oxygen content of the high temperature liquid. The determination can also comprise the measurement of the oxygen content and the temperature.

The parameter of the high temperature liquid is determined with a sensor unit. In other words, the sensor unit is adapted to measure the at least one parameter of the high temperature liquid. It is to be understood, that the sensor unit comprises at least one sensing element; it may comprise further components. The sensor unit is in principle constructed as a disposable component, which dissolves or burns in the high temperature liquid after the parameter of the high temperature liquid is determined. Parts of the sensor unit may dissolve already prior to or during the determination of the parameter.

The sensor unit comprises a sensing element, which can be at least one selected from the group consisting of an electrochemical sensor, an electromagnetic sensor, an optical sensor, a thermoelectric sensor, a sensor for detecting an electrical voltage, a sensor for detecting an electrical current, a sensor for detecting an electrical resistance or a combination of any of the named sensors, so that combined measurements of several parameters are possible.

In particular, the sensor unit can comprise a thermocouple for measuring the temperature of the high temperature liquid and/or an electrochemical cell, preferably an electrochemical cell for determining the oxygen activity of the high temperature liquid.

The electrochemical cell, in particular an electrochemical cell to determine the oxygen activity, may comprise a solid electrolyte tube, which is closed on one end, and which contains a reference material and an electrode at the closed end. Such sensors are for example disclosed in EP 0059222 A1. The electrochemical cell may also be provided as a needle sensor, which comprises a conductive wire which serves as an electrode with at least a solid electrolyte coating and a reference material coating. Such sensors are for example disclosed in US 5332449 A.

The sensor unit may comprise a bath contact. A bath contact is to be understood as an electrically conductive means which provides an electrical contact between the sensor unit and the high temperature liquid. The bath contact may be made from a metal, for example from molybdenum (Mo) or steel. The bath contact may be ring or rod shaped, preferably, the bath contact is ring-shaped. When the sensor unit comprises a thermocouple, such a ring-shaped bath contact preferably surrounds the thermocouple.

A preferred sensor unit comprises a needle sensor to determine the oxygen activity, a thermocouple, preferably a thermocouple enclosed in a quartz sheath, and a ring-shaped bath contact, which surrounds the thermocouple. Such a sensor unit has a compact and robust design, which allows the miniaturization of a measuring probe carrying it.

Preferably, the sensing element of the sensor unit is embedded in an immersion body. Such an immersion body is provided by means of a sheathing in such a manner that the sensing element remains operable for the duration of the measurement. Furthermore, the immersion body aligns the sensor unit once it is immersed such that the sensing element has a suitable orientation and measurement position for the determination of the parameter of interest. Additionally, the immersion body preferably surrounds a connection between the sensing element and a signal line, providing further protection of these sensitive and critical components of the sensor unit. Preferably, the immersion body is made from a heat-insulating material, typically a metal. In preferred embodiments, the immersion body is made from steel, stainless steel or copper.

Preferably, the sensor unit has a weight of less than 1500 g, more preferably less than 1000 g, even more preferably less than 800 g, most preferred less than 500 g. The weight of drop-in sensors used nowadays lies in the range between 4 - 6 kg. The inventive method allows to reduce this weight significantly, reducing the amount of material introduced into the high temperature liquid and additionally lowering production costs due to material savings. Additionally, a measurement is only minimally influenced by the cold mass introduced by the lightweight sensor unit, allowing to obtain reliable and exact data.

Preferably, the sensor unit has a higher density than the density of the high temperature liquid. It has proven to be particularly advantageous when the density of the sensor unit is at least 5 % higher than the density of the high temperature liquid, more preferred at least 8 % higher, even more preferred more than 10 % higher. The density of the sensor unit may be higher than 7,2 g/cm³, more preferably higher than 7,5 g/cm³.

Preferably, the sensor unit does not comprise an additional weight component or floatation component. Due to the high velocity of the sensor unit given by the acceleration means, a further balancing of the sensor unit is not required. Thus, the sensor unit design can be simplified and miniaturized. This miniaturization further especially allows to introduce the sensor unit into metallurgical vessels through available entry points, for which conventional drop-in sensors or lance systems are too large.

Preferably, the sensor unit is configured to be connected to a processing unit. Such a processing unit is configured to process the measured data from the sensor unit and to determine the parameter of the high temperature liquid.

The sensor unit can be covered with a protection element which covers the sensing element during handling and against damages caused by the impact on the high temperature liquid surface. Preferably, the sensor unit is covered with a protective cap, which encloses at least the sensing element, and which is formed from a material that dissolves or melts in the high temperature liquid. It is to be understood, that such a protection element is not part of the sensor unit when it is referred to the density of the sensor unit.

Preferably, the sensor unit comprises a contact element, which is adapted to connect the sensor unit to a suitable means to transfer the measured data, preferably to a signal line. The sensor unit typically comprises an immersion end and a contact end. Advantageously, the contact element is arranged on or in the contact end of the sensor unit. Such a contact element allows an easy mounting of the sensor unit in or at the measuring probe, stabilizes the contact to the signal line and reduces the forces on the sensor unit during acceleration.

The method according to the invention comprises the provision of acceleration means adapted to increase the speed of the sensor unit. In other words, the acceleration means is adapted to shoot the sensor unit in a predetermined direction. Accordingly, the acceleration means is dimensioned, constructed and arranged to accommodate the passage of the sensor unit.

The acceleration means is provided above the surface of the high temperature liquid. In other words, the acceleration means is provided above the highest allowable level of surface of the high temperature liquid. The highest allowable level is the level of the high temperature liquid which is reached when the container is filled with its maximal filling capacity of the high temperature liquid. "Above" in connection with the high temperature liquid in the container means a position higher than the zero level, which is to be found at the bottom of the container. In configurations in which the high temperature liquid is covered with a further layer of material, for example a slag layer, the acceleration means if preferably provided above the further layer of material.

Preferably, the acceleration means is adapted to separate the sensor unit from at least one further component of the measuring probe and accelerate the sensor unit. It is to be understood, that the at least one further component of the measuring probe is not accelerated.

In case the sensor unit is connected by a signal line to a processing unit, for example by cables or wires, the signal line is not in particular accelerated by the acceleration means but is pulled behind the accelerated sensor unit. However, a certain part of the signal line, especially the section, which is directly connected to the sensor unit, will naturally also be accelerated by the acceleration means.

The acceleration means is provided in a distance D_{A} to the surface of the high temperature liquid and the distance D_{A} between the surface of the high temperature liquid is less than 50 % of the distance of the surface of the high temperature liquid to the opening of the container D_{M} (D_{A} < 50 % D_{M}). In other words, the acceleration means is preferably positioned below half the distance of the high temperature liquid to the top opening of the container comprising it.

Previously it was assumed, that a provision of a sensor unit near to the high temperature liquid, i.e. in a device which provides it previous to the introduction into the metallurgical container, could not be realized, since the gravitational speed gained on a significantly shortened travel distance will not be sufficient for a suitable impact through the surface of the high temperature liquid. Surprisingly, it has been found that such a positioning of the acceleration means is not only suitable but even advantageous to conduct the inventive method, even though the distance to the surface of the high temperature liquid is significantly shorter than in classical set-ups. Furthermore, it allows to gain several advantages. A position adjacent to the surface of the high temperature liquid shortens the distance the sensor unit has to travel through the interior of the metallurgical container, which allows to reduce the required amount of protection means and/or material for the sensor unit as well as the length of a required signal line, resulting in a reduced material requirement and thus lower manufacturing costs of the disposable components. Furthermore, the residence time of the sensor unit in the interior of the container before the immersion in the high temperature liquid is reduced, reducing corrosive influences of the high temperature environment.

It can be preferred, that the distance D_{A} is less than 40 % of the distance of the surface of the high temperature liquid to the opening of the container D_{M} (D_{A} < 40 % D_{M}), it can even be preferred, that the distance D_{A} is less than 30 % of the distance of the surface of the high temperature liquid to the opening of the container D_{M} (D_{A} < 30 % D_{M}).

Preferably, the acceleration means is positioned in and/or on a side wall of the metallurgical container providing the high temperature liquid. Thus, the acceleration means is preferably positioned sideways to the surface of the high temperature liquid, in other words, the acceleration means is positioned laterally to the high temperature liquid. Previously it was assumed, that a sideways provision of a sensor unit, i.e. in a device which provides it previous to the introduction into the metallurgical container, could not be realized, since the demanding environment at these positions would not be suitable as an access point. Surprisingly, it has been found that a sideways positioning of the acceleration means is not only suitable but even advantageous to conduct the inventive method, even though the distance to the surface of the high temperature liquid is significantly shorter than in classical set-ups.

The acceleration means may extend into or adjoin to the volume of the metallurgical container containing the high temperature liquid through an opening in the side wall of the metallurgical container. In other words, the acceleration means preferably extends through the side wall of the container. Especially in cases where no access from a vertical position above the container or from a slag door is feasible the positioning of an acceleration means in a sideway position to the bath is advantageous. This is especially advantageous in EAF facilities, which are covered by a removable lid and comprise electrodes which are introduced through this cover.

It may be preferred that the acceleration means is installed in a removable manner. In cases where the container is a metallurgical vessel, the acceleration means can be anchored in an insulating layer of the side wall when it is provided.

The acceleration means may be oriented downwards towards the high temperature liquid through a side wall of the metallurgical container. In preferred embodiments, the angle between the acceleration means and an inner surface of the side wall towards the high temperature liquid is at least 25°, more preferred at least 30°, even more preferred at least 35°. Preferably, the angle is in the range between 25 and 75°, more preferred between 35 and 65°.

In preferred embodiments, the metallurgical container is an EAF which comprises a slag door and the acceleration means is not installed in, at or on the slag door.

The acceleration means typically comprises a loading end with a loading opening and a forward end with an exit opening. The loading opening is adapted to insert the measuring probe carrying the sensor unit. It is to be understood, that the acceleration means is thus configured to accommodate the measuring probe carrying the sensor unit. The exit opening is adapted for the sensor unit to exit the acceleration means after acceleration. Preferably, the distance between the loading opening and the exit opening is shorter than 2 m, more preferred shorter than 1,5 m, even more preferred shorter than 1,3 m. The distance between the loading opening and the exit opening can for example be in the range of 0,3 to 1,5 m, preferably in the range of 0,5 to 1,3 m.

The acceleration means is configured to accelerate the sensor unit along an acceleration path that extends between the loading end and the exit opening. In other words, the acceleration path is the path the sensor unit is accelerated along inside the acceleration means during acceleration. The acceleration path typically does not begin at the loading end, since the measuring probe carrying the sensor unit and the sensor unit itself may have a certain length, positioning the sensor unit in the direction of the exit opening prior to acceleration in a distance to the loading end. In other words, the acceleration path begins at the position of the sensor unit when it is provided to the acceleration means and after separation from the measuring probe. In preferred embodiments, the acceleration path is straight. Preferably, the acceleration path is positioned parallel to a central longitudinal axis of the acceleration means along its length, more preferably the acceleration path is positioned coaxial to the central longitudinal axis. In other words, the sensor unit is preferably accelerated centrally inside the acceleration means. Preferably, the length of the acceleration path is at least 0,2 m, more preferred at least 0,4 m. The length of the acceleration path can for example be in the range of 0,2 to 1,4 m, preferably in the range of 0,4 to 1,2 m.

Preferably, the acceleration means comprises a hollow elongated member, for example a tube. Preferably, the acceleration means comprises a tube. In such cases, the acceleration path is preferably positioned parallel to the central longitudinal axis of the hollow elongated member.

After acceleration, the sensor unit can be ejected from the hollow elongated member through the exit opening.

The forward end of the acceleration means can be flush with the inside of the side wall of the container, it may also extend out of the side wall and thus be positioned in the volume of the container.

The forward end may comprise a nozzle. In one embodiment, an end of the acceleration means which is directed or directable towards the high temperature liquid is realized as a de Laval nozzle. This enables a purge gas flow to be introduced into the metallurgical container at a high speed and/or supersonic speed.

The acceleration means can be operated by any mechanism known to the skilled person, for example by a pneumatic mechanism, a hydraulic mechanism, a mechanic mechanism like a pushing mechanism or a spring, in particular a preloaded spring, or an electromagnetic mechanism. In preferred embodiments, the acceleration means comprises pneumatic means configured to accelerate the sensor unit, such means may comprise a compressed gas supply.

In case of a pneumatic mechanism, a gas flow is used to accelerate the sensor unit. The term gas in the context of the invention refers to any gaseous material, e.g. a gas, a gas mixture and/or a dispersion having gas as continuous medium. Thus, a gas flow may be a flow of mixture of gases such as air. The acceleration means can in particular be operated by pressurized air or nitrogen. Typically, gas lines are highly temperature resistant which renders them particularly suitable for the use in high temperature environments. Pneumatically driven acceleration means are further advantageous, since only minimal equipment is necessary to be installed near the point of operation and the installation only requires mechanical parts which can be designed in a robust way. Thus, an installation which is low in maintenance demands can be utilized. Furthermore, and especially in metallurgical containers, such a gas flow can additionally be used to prevent slag ingress that could cause clogging of the acceleration means.

In embodiments with a pneumatic mechanism, the acceleration means is preferably provided with an inlet or coupling for introducing a flow of gas, preferably a flow of pressurized gas, into the interior of the acceleration means. The gas flow is then discharged through the exit opening during operation. The acceleration device is preferably connected to at least one gas line for connecting a high-pressure gas source in order to generate a gas flow in the acceleration means along the acceleration path. Preferably, the inlet or coupling for introducing a gas flow is positioned laterally on the acceleration means.

The acceleration means may comprise a second inlet or coupling for introducing a second flow of gas. Such a second flow of gas may be provided as a purge gas flow, which can be applied permanently during the processing operation of a metallurgical vessel. The purge gas flow may serve to keep the hollow space within the acceleration means free of debris from the high temperature liquid and ensures a reliable operation.

In preferred embodiments, the acceleration means is purged permanently with the purge gas flow and during acceleration of the sensor unit an additional flow of gas is applied. Thus, the use of pressurized gas can be minimized.

Preferably, the acceleration means is made of a suitable temperature resistant material, for example a ceramic material, a metal or an alloy. Preferably, the acceleration means is made from steel or stainless steel.

The acceleration means may comprise means for releasing the sensor unit from the rest of the probe in cases where the sensor unit is provided within such a probe, for example an ejector for releasing the sensor unit or a mechanical means which is configured to separate the sensor unit from the carrying element of the measuring probe.

During metallurgical processing, chemicals, typically in particular form, such as lime, calcium, carbon, oxygen, aluminum, and silicon, may be introduced into the molten metal to alter the chemical composition of the metal. Means for such an insertion can for example be a blowing lance or an injection system used to actively treat the high temperature liquid with additives in particulate or gaseous form. A blowing lance is a lance through which a flow of gas can be blown into the metallurgical container. The gas flow may comprise further elements for the treatment of the molten metal. Additionally, means to introduce chemical energy in form of fluids, in particular gases, are typically provided to heat and/or treat the molten metal, for example burners or oxygen injectors. Such means are often permanently or removably installed at the metallurgical container and are introduced through an opening which is usually positioned just above the level of the molten metal. To ensure that the opening towards the molten metal is kept open, such installations are purged with a permanent flow of gas, mostly compressed air or nitrogen.

In preferred embodiments, the acceleration means is provided in or on an insertion means adapted to insert fluid and/or particles into the high temperature liquid. In other words, the acceleration means can be provided as a means with dual function. This can help prevent penetration of metal, slag and/or debris into the feeding channel of the injecting means and/or the acceleration means. Thus, the installation of the acceleration system can be facilitated in a space-saving manner and furthermore reduces installation costs.

Especially the installation of a pneumatically driven acceleration means in or on an insertion means allows a favorable installation, which requires a minimal number of extra components with only a small footprint within the limited space available in a metallurgical facility. In vicinity to the container, no movable parts need to be present, reducing the risk of time-consuming incidents.

The method according to the invention comprises providing a measuring probe carrying the sensor unit to the acceleration means. It is to be understood, that "providing the measuring probe to the acceleration means" comprises the loading of the measuring probe to or in the acceleration means. In other words, after this step, the acceleration means is loaded and/or in contact with the measuring probe.

A measuring probe is to be understood as a device which is configured to provide a sensor unit and/or a sensing element before its use. In other words, the measuring probe comprises the sensor unit and at least one additional element. For example, such a probe may comprise a protection element to carry and/or protect the sensor unit during transportation and storage, a connection element for electrical and/or mechanical connection and/or means to transfer a signal generated by the sensor unit as a signal line.

The sensor unit is a separable from the measuring probe. In other words, the sensor unit is separable from at least one additional element of the measuring probe. For example, the at least one further element of the measuring probe may comprise a first coupling component which is configured to releasably engage a second coupling component arranged on or at the sensor unit. The at least one additional element of the measuring probe may also comprise a catch element, which releases the sensor unit upon the application of a certain force.

Preferably, the measuring probe comprises a carrier element, preferably a carrier tube, for example a cardboard tube, which can at least partly accommodate the sensor unit and optionally further elements of the measuring probe. Preferably, the measuring probe does not comprise elements which extend laterally from the carrier element.

Preferably, the measuring probe comprises at least one signal line which is configured to connect the sensor unit to a processing unit. The signal line may comprise one or more wires or cables. In such cases, the measuring probe advantageously comprises a probe contact element configured to be connected to the at least one signal line.

The at least one signal line may be wound up within the measuring probe carrying the sensor unit, in particular within a tube of the measuring probe. For example, the signal line or lines can run through the inside of a carrier tube and are wound up inside of the carrier tube around its longitudinal axis.

Alternatively, the sensor unit may comprise means suitable for wireless transmission of the measured data to a processing unit. In such cases, the sensor unit is adapted to transmit the measured data to a wireless data signal receiver by radio frequency signals or the like.

Prior to the provision to the acceleration means, the measuring probe can be stored individually; it may also be stored in a storage unit containing multiple measuring probes. Such storage units make it particularly possible to trigger measurement cycles automatically without any manual intervention with the system carrying out the inventive method. Thus, a storage unit further enhances the autonomy of a such a system since the system may be loaded with several probes at the same time and necessary further interaction is minimized.

The method according to the invention comprises separating the sensor unit from the measuring probe. In other words, the sensor unit is released from the further components of the measuring probe prior to the acceleration.

This separation may for example be realized by a releasing mechanism provided with the measuring probe and/or corresponding releasing means provided by the acceleration means. The separation may also be conducted by external separating means.

The method according to the invention comprises the acceleration of the sensor unit with the accelerating means. During acceleration, the sensor unit is accelerated along the acceleration path of the acceleration means.

The sensor unit is accelerated with an acceleration. As known to the skilled person, the acceleration is the rate of change of the velocity of an object with respect to time. The acceleration is to be understood as the average acceleration along the acceleration path. Preferably, the acceleration is at least 15 m/s², more preferred at least 20 m/s², even more preferred at least 25 m/². In preferred embodiments, the acceleration lies in the range of 15 - 80 m/s², preferably in the range of 25 - 70 m/s². Such an acceleration allows even lightweight sensor units with a weight of less than 1500 g or even less than 1000 g to be sufficiently accelerated.

It has been proven advantageous, when the acceleration path has a length between 0,2 - 1,4 m and the acceleration lies in the range of 15 - 80 m/s². Such a configuration allows even lightweight sensor units to be sufficiently and efficiently accelerated.

The acceleration may or may not be constant along the acceleration path, for example, the acceleration may comprise a rising phase with an increase in acceleration and a constant phase with a constant acceleration. The acceleration may comprise more than one acceleration, for example the acceleration may comprise a first acceleration and a second acceleration, preferably the first acceleration is smaller than the second acceleration.

Preferably, the exit speed of the sensor unit is at least 3 m/s, more preferred at least 5 m/s, even more preferred more than 8 m/s. In advantageous embodiments, the exit speed of the sensor unit lies in the range of 3 - 18 m/s, more preferred in the range of 5 - 15 m/s. The exit speed is to be understood as the speed the sensor unit has when it is projected from the accelerating means. A minimal exit speed has been shown especially advantageous for lightweight senor units, for example with a weight lower than 1500 g or even lower than 1000 g. A minimal exit speed allows such sensor units to gain a sufficiently high impact to enter the surface of the high temperature liquid.

In preferred embodiments, the acceleration means comprises an acceleration path shorter than 1,5 m and the sensor unit is accelerated to a speed of at least 5 m/s. Such a configuration allows even lightweight sensor units to have a high enough impact when reaching the surface of the high temperature liquid.

In preferred embodiments, the sensor unit has a weight of less than 1000 g, more preferred less than 500 g, and is accelerated to a speed of at least 5 m/s. Such a combination allows even these lightweight sensor units to have a sufficiently high impact when reaching the surface of the high temperature liquid.

After acceleration, the sensor unit will have obtained a certain momentum. As known to the skilled person, the momentum is the product of the mass and velocity of an object. Preferably, the sensor unit is accelerated to obtain a momentum of at least 1000 g*m/s, more preferred at least 1500 g*m/s, even more preferred at least 2000 g*m/s. In preferred embodiments, the momentum is in the range of 1000 - 10000 g*m/s, preferably in the range of 1500 - 8000 g*m/s. A minimal momentum allows even lightweight sensor units to gain a sufficiently high impact to enter the surface of the high temperature liquid.

In a preferred embodiment, the sensor unit has a weight of less than 1000 g and is accelerated to obtain a momentum of at least 1000 g*m/s. Such a combination allows even these lightweight sensor units to have a sufficiently high impact when reaching the surface of the high temperature liquid.

The method according to the invention comprises projecting the sensor unit in the direction of the high temperature liquid. "Projecting the sensor unit" means, that the sensor unit is released from the acceleration means. Subsequently, the sensor unit moves towards the high temperature liquid. During this movement, the sensor unit travels along a projection trajectory.

Preferably, the sensor unit is projected with an angle of at least 25° relative to a surface normal of the high temperature liquid, more preferred at least 30°, even more preferred at least 35°. The surface normal of the high temperature liquid is to be understood as an axis perpendicular to the surface. It is to be understood, that the angle of projection is mainly determined by the position and orientation of the acceleration means relative to the side wall of the metallurgical container. In configurations of metallurgical containers with side walls arranged perpendicular to the surface of the high temperature liquid, the surface normal is aligned parallel to the side walls. Preferably, the angle of projection is in the range between 25 and 75°, more preferred between 35 and 65°. The projection with a certain angle relative to the surface normal or the side wall allows the sensor unit to reach a certain distance from the side wall before entering the high temperature liquid and with this a typically more homogeneous area where more reliable and representative measurement results can be obtained.

Preferably, the projection trajectory is straight, in other words, the projection trajectory is not curved. A straight projection trajectory allows the shortest length of the trajectory and with this minimizes the travel time of the sensor unit. It may be preferred, that the projection trajectory has a curvature of less than 5 °, more preferred less than 3 °.

When the sensor unit is connected with a signal line, for example by means of a wire, wires or a cable, the signal line is pulled by the sensor unit and follows its movement. In cases where the signal line is wound in the measuring probe, it is unwound during this phase.

The method according to the invention comprises immersing the sensor unit under the surface of the high temperature liquid. In other words, the sensor unit enters the high temperature liquid after the phase during which it moves with at least the speed provided by the acceleration means towards the high temperature liquid. In cases of molten metals with a present slag layer, the sensor unit will move through this slag layer before entering the molten metal.

The sensor unit enters the surface of the high temperature liquid at a point of impact in a distance D_{I} to the entry point in the container. Preferably, this point of impact is positioned in a distance smaller than half of the diameter of the container (D_{V}), in other words D_{I} < 50 % D_{V}, even more preferred, D_{I} < 30 % D_{V}. An impact in proximity to the side wall of the container is advantageous, since the distance the sensor unit has to travel within the vessel is minimized.

Preferably, the sensor unit is immersed with an angle of less than 65° relative to the surface of the high temperature liquid, more preferred with an angle less than 60°, even more preferred with an angle less than 55°. Drop-sensors typically pass the molten metal surface with an angle of immersion of about 90°. A smaller immersion angle lowers the resisting forces of the high temperature liquid surface during the immersion phase, thus the sensor unit may be less prone to damages by the impact. Especially in metallurgical applications where a slag layer needs to be passed before the immersion under the surface of the molten metal, a lower immersion angle is favorable for the operability of the sensor unit. The sensor unit may for example enter the high temperature liquid with an immersion angle between 15 to 65°, preferably with an immersion angle between 25 to 55°, more preferred with an immersion angle between 30 to 50°. An immersion angle in the preferred range allows a position of the point of impact in a sufficient distance to the side wall while simultaneously minimizing the distance of the point of impact, which minimizes the required signal line length and enables a sufficient diving depth of the sensor unit. Furthermore, when the immersion angle becomes too small, the distance the sensor unit needs to travel through an additional layer of material increases, which may decrease the speed too far prior to the impact on the surface of the high temperature material.

The method according to the invention comprises measuring the at least one parameter of the high temperature liquid. It is to be understood, that the measurement is taken when the sensor unit is immersed under the surface of the high temperature liquid. "Measuring" is used herein to describe a step resulting in the determination of the at least one parameter of the high temperature liquid. This step may comprise the measurement of a single data point or the measurement of more than one data point; i.e. the measurement of a series of data points.

The measuring may comprise further steps, for example the transmittance of data to a processing unit and/or the processing of the data.

After passing the surface of the high temperature liquid, the sensor unit dives to a certain depth. During this diving phase, the speed gradually decreases since the sensor unit is slowed down by the surrounding high temperature liquid. During this phase, the sensor unit will be heated up by the high temperature liquid. Ultimately, the speed in the forward direction will be zero, in other words the movement of the sensor unit will stop. When the weight and density of the sensor unit are chosen accordingly, a rising phase will follow, during which the sensor unit will move in the upward direction again. Preferably, the measurement is conducted during this rising phase. Thus, the recorded data will arise from parts of the high temperature liquid, which have previously not been influenced by the sensor unit, providing more accurate data.

After the measuring of the parameter of the high temperature liquid, further steps may follow, for example the release or ejection of parts of the measuring probe which remained within or at the acceleration means after the acceleration of the sensor unit, the cutting of remaining cables or the like.

The invention also refers to a system to carry out the inventive method. All embodiments described in relation to the inventive method may also apply in any combination to the inventive system.

Preferably, the system comprises acceleration means.

In preferred embodiments, the acceleration means is configured to accelerate a sensor unit of less than 1000 g to obtain a momentum of at least 1000 g*m/s.

In preferred embodiments, the acceleration means is configured to accelerate a sensor unit of less than 1000 g, more preferred less than 500 g, to a speed of at least 5 m/s over an acceleration path shorter than 1,5 m.

Preferably, the system according to the invention comprises a processing unit. A processing unit is to be understood as a unit configured to obtain and process a signal obtained by a suitable sensor unit to determine the parameter of interest of the high temperature liquid. In preferred embodiments, the processing unit is configured to be arranged outside of the metallurgical container containing the high temperature liquid.

Preferably, the system comprises means to transfer a signal obtained by a suitable sensor unit. These means may for example comprise an extension wire which is configured to be connected to the sensor unit and the processing unit, also means for wireless transfer may be suitable.

The system may also comprise a loading unit to load a measuring probe into the acceleration means.

The system may further comprise a storage unit, in which measuring probes can be stored before their use.

The invention also refers to a metallurgical vessel comprising a system to carry out the inventive method. All embodiments described in relation to the inventive method, or the inventive system may also apply in any combination to the inventive metallurgical vessel.

Preferably, the metallurgical vessel is an electric arc furnace.

The following schematic drawings show aspects of the invention for improving the understanding of the invention in connection with some exemplary illustrations. It should be understood, however, that the invention is not limited to the precise arrangements and instrumentalities shown. The elements of the drawings are not necessarily to scale relative to each other. Like reference numerals designate corresponding similar parts. Herein
- Figure 1: shows a schematic cross-sectional view of an exemplary measuring probe;
- Figure 2: shows a schematic metallurgical vessel having an acceleration means installed in a side wall;
- Figure 3: shows an acceleration and a speed profile of an exemplary measurement sequence for the measurement in a high temperature liquid which is not covered with an additional layer of material;
- Figure 4: shows an acceleration and a speed profile of an exemplary measurement sequence which is covered with an additional layer of material;
- Figure 5: shows a schematic cross-sectional view of a container comprising a high temperature liquid with a sideways arranged accelerator.

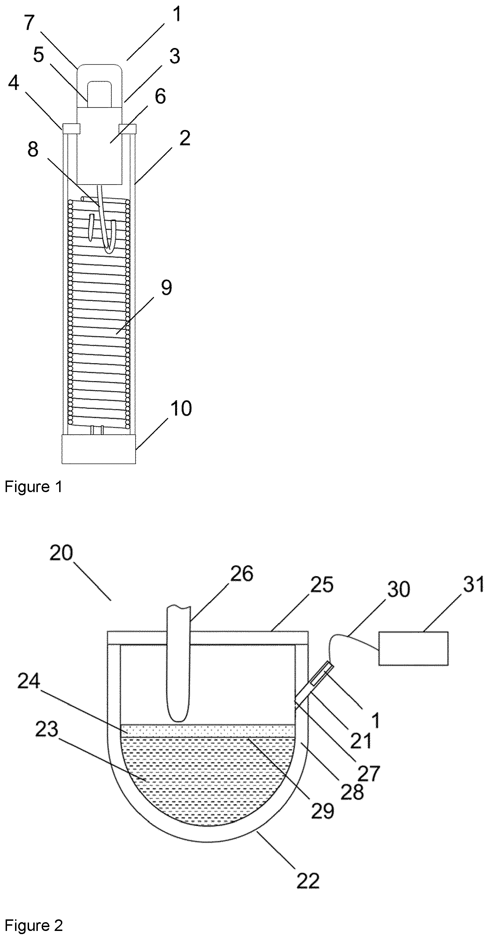

Fig. 1 shows a schematic cross-sectional view of an exemplary measuring probe 1. The probe 1 comprises a carrier tube 2 which may be formed from cardboard. A sensor unit 3 is mounted at least partly within the carrier tube 2 at one end and is held by a releasing mechanism 4. The sensor unit 3 comprises a sensing element 5 and an immersion body 6. The sensing element 5 is chosen according to the parameter to be measured, for example it may comprise an oxygen detecting element and/or a temperature measurement element like a thermocouple. The immersion body 6 is preferably a solid metal body with a high density and high thermal conductivity, for example a solid steel body with a bore to engage the sensing element 5. To protect the sensing element 5 during the handling of the measuring probe 1, a protective cap 7 formed from a material that dissolves or melts in the respective high temperature liquid encloses the sensing element.

Along the inside of the carrier tube 2 a signal line 8, which is connected to the sensor unit 3 on one end, is wound up in windings 9 along the inside of the carrier tube 2. The connection may be realized by a contact piece which is arranged in the immersion body 6 (not shown). The other end of the signal line is connected to a connection element 10 at the other end of the carrier tube. The connection element 10 may provide a suitable connection point to an extension cable or means to wirelessly transfer a signal acquired by the sensor unit to an analyzing unit.

Fig. 2 shows a schematic metallurgical vessel 20 like an electrical arc furnace (EAF) having an acceleration means (an accelerator, 21) integrated in a side wall. An EAF used for steelmaking usually comprises a container 22 containing the molten metal bath 23 and a removable lid 25 through which one or more electrodes 26 can enter the furnace. A slag layer 24 covers the molten metal 23. The electrodes 26 employed to heat the metal are arranged above the container 22. Typically, the interior of the metallurgical vessel 20 is heated to temperatures of about 600 - 2000 °C or even higher, during processing.

An entry point 27 which is usually used for installations for the treatment of the molten metal bath, such as a carbon injector, is placed in a side wall 28 of the container 22. This entry point 27 may also serve to accommodate the accelerator 21, preferably the accelerator 21 can be combined with a means to treat the molten metal bath 23. The accelerator 21 can for example be an elongated tube like a pneumatically driven blowing lance. Advantageously, such pneumatic devices can be permanently purged with a gas stream which keeps the entry point and the opening of the accelerator open. In a preferred embodiment, the accelerator comprises a vacuum conveyer (e.g. commercially available for example from Sommer Technik GmbH, Straubenhardt, Germany) mounted on a steel tube. In an exemplary embodiment, the inner tube of the accelerator has a length of 1,5 m. When a probe is provided, the sensor unit is positioned in a distance of 1,3 m to the opening of the accelerator which is orientated towards the molten metal bath 23. To accelerate the sensor unit, a gas flow of 3200 l/min is applied, which leads to an exit speed of 10 m/s for a sensor unit of 200 g.

The accelerator 21 traverses through the side wall 28 of the container with its tip arranged flush with the interior of the side wall 28. The accelerator 21 is arranged in such a way that a sensor unit projected from the accelerator 21 after an acceleration phase enters the surface of the melt 29. The accelerator 21 may also be surrounded by a so called cold-box, which is an entity arranged at the interior of the vessel to provide protection for the encased devices. Typically, parts of the vessel interior not in contact with the molten metal bath are supplied with a cooling mechanism, for example a water cooling.

In the shown configuration of Fig. 2, the accelerator 21 is loaded with a measuring probe 1, which carries a suitable sensor unit (not shown). An extension cable 30 connects the sensor unit to a processing device 31, which may be placed in a distance to the vessel.

In a typical measurement sequence, the accelerator is loaded in a first step with the measuring probe. Inside the accelerator, the sensor unit is separated from the carrier parts of the probe. This separation may for example be realized by a suitable installation inside the accelerator, like a shoulder or a barrel shaped cone, against which a holding means of the probe is pushed to release the sensor unit. It shall be emphasized, that any connections between the sensor unit and a signal line or suitable connectors are not released and are all configured to remain in place at least until the measurement sequence is finished.

Subsequently, the sensor unit is accelerated, for example by compressed air, and ejected from the accelerator 21 into the interior of the vessel 22 and towards the molten metal bath 23 with a high initial speed and momentum. The sensor unit flies on a straight path towards the molten metal and enters the surface 29. A signal line which is connected to the sensor unit will be pulled behind the sensor unit and out of the carrying elements of the probe and is chosen to survive the circumstances inside the vessel long enough to ensure that the measurement can be taken.

When the sensor unit is immersed under the surface of the molten metal bath, the desired parameter can be measured, and the respective signal is transferred to a suitable analyzing device. After the recording of the required data, the accelerator may be cleared from the elements of the probe which have not been projected into the molten metal, for example by ejecting them into the molten metal bath.

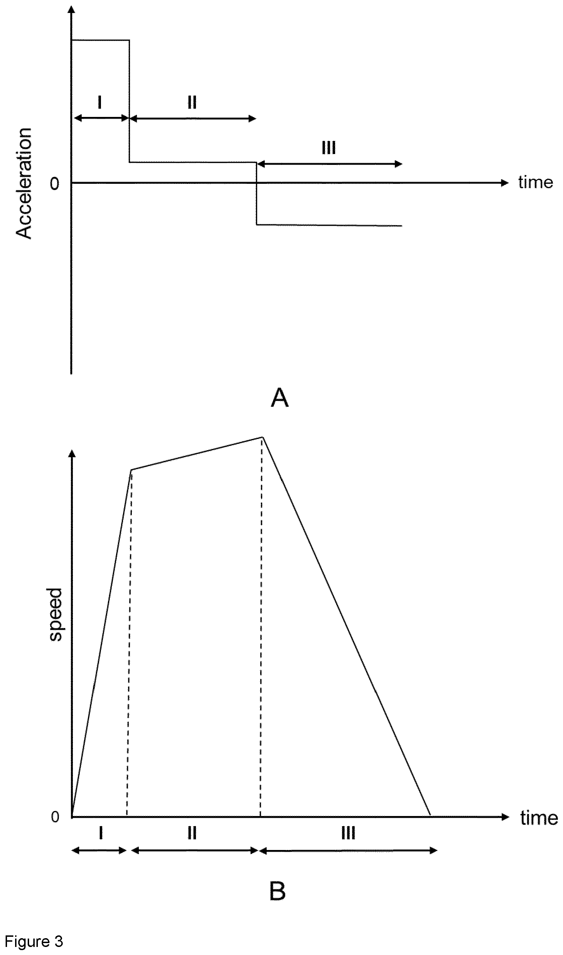

Fig. 3 shows an acceleration (A) and a speed profile (B) of an exemplary measurement sequence for the measurement in a high temperature liquid which is not covered with an additional layer of material. The profiles start after the sensor unit has been separated from the immersion probe and covers the phases during which the sensor unit is accelerated and/or in motion. In a first phase (I), the sensor unit is actively accelerated by the accelerator with an acceleration significantly higher than gravity. It is to be understood, that the shown constant acceleration refers to the average acceleration during this phase, it may comprise several acceleration phases or phases with an increase or decrease in acceleration. Accordingly, the shown constantly increasing speed of the phase I is also to be understood as an average and may also comprise more than one phase. It has been shown that a high exit speed of at least 5 m/s is required for a sensor unit of less than 1000 g to allow for a sufficient impact through the surface of the high temperature liquid. Subsequently, the sensor unit is ejected from the accelerator with this exit speed. In the following "free-flying" phase (II), gravity further accelerates the sensor unit and the travelling speed increases. After the impact through the surface of the high temperature liquid, the sensor unit is de-accelerated by the opposing forces of the liquid. In this "diving phase" (III) the speed decreases until it reaches zero. At this point in time, the sensor unit has reached its final measuring position deep enough under the surface to obtain reliable results in a homogeneous area of the high temperature liquid.

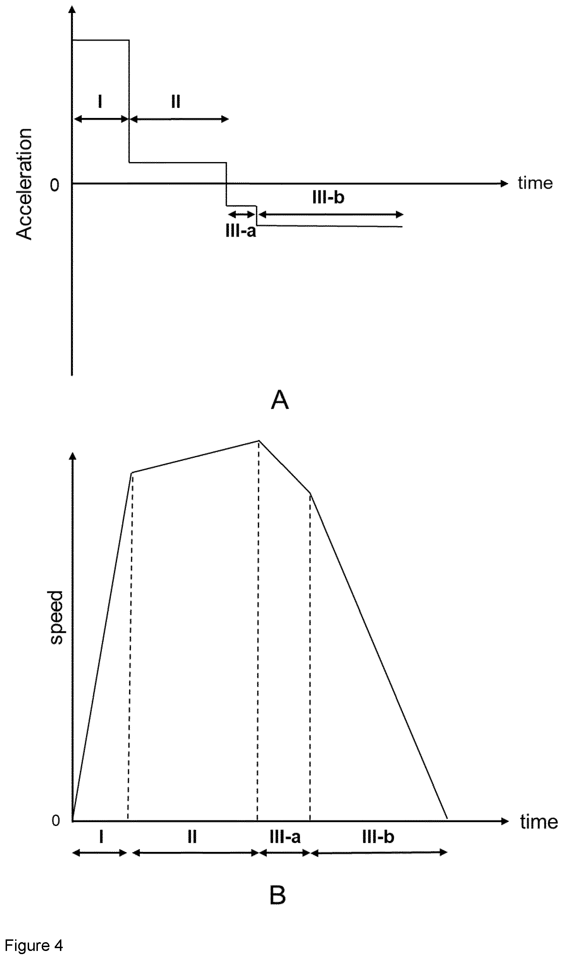

Fig. 4 shows an acceleration (A) and a speed profile (B) of an exemplary measurement sequence which is covered with an additional layer of material like a slag layer. Prior to the impact in the high temperature liquid, the sensor unit passes the additional layer which has a deaccelerating effect and speed-reducing effect (phase III-a).

Due to the high momentum the sensor unit is provided by the active acceleration, it dives deep into the molten metal despite its low mass, the short distance between the entry point and the surface and a slag layer which deaccelerates the unit before the final immersion in the molten metal. Additionally, the measurement is minimally influenced by the cold mass introduced by the lightweight sensor unit, allowing to obtain reliable and exact results.

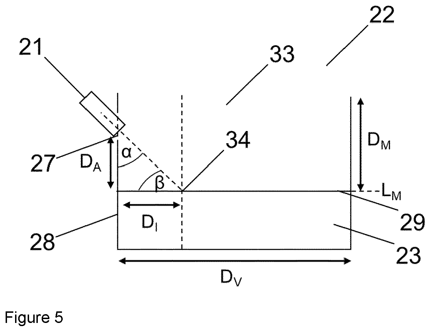

Fig. 5 shows a schematic cross-sectional view of a metallurgical container 22 comprising a molten metal bath 23 with a sideways arranged accelerator 21 with the relevant geometrical parameters indicated. The accelerator 21 is positioned in a distance D_{A} to the level L_{M} of the surface of the molten metal, which has a distance D_{M} to the opening of the container 33 The container has a diameter D_{V}. After the free-flying phase, the sensor unit enters the molten metal at a point of impact 34 on its surface, which has a distance D_{I} to the entry point 27 in the side wall 28. The angle of injection, which is the angle between the normal to the surface of the high temperature liquid and the trajectory of a sensor unit ejected by the accelerator is depicted with α. The angle of injection is also the angle of projection, i.e. the angle of the projection trajectory of the sensor unit relative to the surface normal of the high temperature liquid (indicated by a dashed line). The immersion angle β is the angle between the surface of the high temperature liquid and the trajectory of the sensor unit.

### Reference Signs

- 1: measuring probe
- 2: carrier tube
- 3: sensor unit
- 4: releasing mechanism
- 5: sensing element
- 6: immersion body
- 7: protective cap
- 8: signal line
- 9: windings of signal line
- 10: connection element

- 20: metallurgical vessel
- 21: accelerator
- 22: container
- 23: molten metal bath
- 24: slag layer
- 25: removable lid
- 26: electrode
- 27: entry point
- 28: side wall of container
- 29: surface of molten metal bath
- 30: extension cable
- 31: processing device
- 32: high temperature liquid
- 33: opening of container
- 34: point of impact

- L_{M}: Position of surface level of high temperature liquid
- D_{A}: Distance acceleration means to surface of high temperature liquid
- D_{M}: Distance of opening of container to surface of high temperature liquid
- D_{V}: Diameter of container
- D_{I}: Distance point of impact to entry point

- α: angle of injection / angle of projection
- β: immersion angle

## Claims

1. A method for determining at least one parameter of a high temperature liquid with a sensor unit,
wherein the high temperature liquid comprises a surface and is provided in a metallurgical container which comprises a top opening opposite the surface of the high temperature liquid, and
the surface of the high temperature liquid level has a position L_{M}, which is positioned in a distance D_{M} to the top opening of the metallurgical container,
the method comprising
(a) providing acceleration means above the surface of the high temperature liquid in a distance D_{A} between the surface of the high temperature liquid and the top opening of the metallurgical container, wherein the acceleration means is adapted to increase the speed of the sensor unit;
(b) providing a measuring probe to the acceleration means, wherein the measuring probe carries the sensor unit and wherein the sensor unit is separable from the measuring probe;
(c) separating the sensor unit from the measuring probe;
(d) accelerating the sensor unit with the acceleration means;
(e) projecting the sensor unit in the direction of the high temperature liquid;
(f) immersing the sensor unit under the surface of the high temperature liquid;
(g) measuring the at least one parameter of the high temperature liquid;
**characterized in that** D_{A} < 50 % D_{M}.

2. A method according to claim 1, wherein the acceleration means is provided in and/or attached to a side wall of the metallurgical container.

3. A method according to claim 1 or 2, wherein the acceleration means extends into or adjoins to the volume of the metallurgical container containing the high temperature liquid through an opening in a side wall of the metallurgical container.

4. A method according to any of the preceding claims, wherein the acceleration means is oriented downwards towards the high temperature liquid through a side wall of the metallurgical container.

5. A method according to any of the preceding claims, wherein the sensor unit is immersed under the surface of the high temperature liquid with an immersion angle smaller than 65°.

6. A method according to any of the preceding claims, wherein the sensor unit is projected with an angle of more than 25° relative to a side wall of the container.

7. A method according to any of the preceding claims, wherein the sensor unit is accelerated to obtain a momentum of at least 1000 g*m/s.

8. A method according to any of the preceding claims, wherein the sensor unit has a weight of less than 1500 g.

9. A method according to any of the preceding claims, wherein the sensor unit is accelerated to a speed of at least 5 m/s.

10. A method according to any of the preceding claims, wherein the acceleration lies in the range of 15 - 80 m/s².

11. A method according to any of the preceding claims, wherein the projection trajectory of the sensor unit after projection and prior to immersion is linear.

12. A system to carry out the method according to claims 1-11.

13. Metallurgical vessel comprising a system to carry out the method according to claims 1 - 11 or a device according to claim 12.
